# EUROPEAN PATENT APPLICATION

(11) **EP 0 580 161 A1**
(43) Date of publication of application: **26.01.1994**
(21) Application number: 93111748.5
(22) Date of filing: 22.07.1993
(51) Int. Cl.: A61K 37/64

(54) **Prophylactic and therapeutic treatment of Alzheimer's disease**

(30) Priority: 22.07.1992 US 925594; 14.08.1992 US 930647
(71) Applicant: THE McLEAN HOSPITAL CORPORATION, Belmont, MA 02178 (US)
(72) Inventor: Saito, Ken-ichi, Midori-ku, Yokohama-shi, Kanagawa-ken (JP); Nixon, Ralph Angus, Arlington, Massachusetts 02174 (JP)
(74) Representative: Hansen, Bernd, Dr. Dipl.-Chem.

(57) **Abstract**

A therapeutic or prophylactic agent for treating Alzheimer's disease which comprises as an essential ingredient a calpain inhibitor is provided.

## Description

The present invention relates to a prophylactic and therapeutic treatment of Alzheimer's disease, which comprises administering a calpain inhibitor to a patient suffering from, or being susceptible to, Alzheimer's disease. The invention also relates to the use of the calpain inhibitor in preparing a pharmaceutical formulation for prophylactic or therapeutic treatment of Alzheimer's disease. Further, the invention relates to said pharmaceutical formulation containing the calpain inhibitor as an active ingredient.

Alzheimer's disease or Alzheimer syndrome is a progressive presenile dementia occurring at age between 45-65. Pathologically, many senile plaque and neurofibrillary changes are observed in the brain of a patient suffering from the disease. Since senile dementia due to natural senescence observed in patients after 65 years old shows no substantial pathological difference from that of Alzheimer's disease, it is called senile dementia of Alzheimer type. The number of patients suffering from these diseases has been increasing with the increase of senile population, and therefore, the diseases have become serious social problem. However, the etiology of this disease is not known despite of the existence of many hypotheses, and early solution for this problem has been desired.

It is known that the incidence of the above-mentioned two pathological changes characteristic to Alzheimer's disease and senile dementia of Alzheimer type (hereafter, the term "Alzheimer's disease" includes both of these dementia) shows a good correlation with the degree of cognitive impairment. Therefore, many studies have been done since the first half of 1980's to throw light on the cause of this disease through study at molecular level of an insoluble cumulative material which causes the above-mentioned two pathological changes.

It is known that disappearance of synapse due to neuronal cell death shows an extremely good correlation with the extent of mental dysfunction (Annals of Neurology, 30, 572 (1991); Annals of Neurology, 39, 355 (1989); Annals of Neurology, 27, 457 (1990)). Mechanism through which this disappearance occurs is not known at molecular level. However, the role of calcium has drawn increasing attention, because it has been reported that the dynamic equilibrium of intracellular calcium is destroyed in Alzheimer's disease as well as in other disorders such as excitatory amino acid toxication, β-amyloid neurotoxication, and free radical disorder (Aging, 1, 17 (1989); Neuron, 1, 623 (1989); Journal of Neuroscience, 12, 376 (1992)). Similar phenomenon has been observed in fibroblasts of patients of Alzheimer's disease (Brain Research, 543, 139 (1991); New England Journal of Medicine, 312, 1063 (1985); Proc. Natl. Acad. Sci. USA, 83, 2758 (1986); Brain Research Bulletin, 21, 825 (1988); Neuroscience Letters, 121, 239 (1991); Biological Psychiatry, 22, 1079 (1987); Brain Research, 565, 42 (1991); Journal of Neuroimmunology, 33, 167 (1991)). However, it was difficult to detect the abnormality of calcium dynamic equilibrium in human brain because the calcium level changes after death.

It appears that the change in calcium dynamic equilibrium affects Ca(calcium)-dependent neutral protease (calpain). This enzyme is believed to participate in the control of intracellular transmission system (Proc. Natl. Acad. Sci. USA, 87, 3705 (1990); Cell Structure Function, 15, 1 (1990); Biochemistry International, 18, 263 (1989)). It has been suggested that restricted degradation by calpain is involved in the control of other important enzymes such as Ca-dependent protein-phosphorylating enzymes, protein-dephosporylating enzymes, and neurotransmitter-related enzymes, and of functions of membrane-constituting proteins and membrane-skeletal proteins (Annals of the New York Academy of Sciences, 568, 198 (1989)). Thus, abnormal activation of calpain appears to give serious effects through various metabolic pathways. While rapid activation of calpain which occurs during ischemia causes an acute neuronal cell death (Journal of Neuroscience, 9, 1579 (1989); Proc. Natl. Acad. Sci. USA, 88, 7233 (1991)), continuous activation at low level appears to have slow but progressive effects on protein-phosphorylation and protein's metabolic turnover. For example, it participates in processing of amyloid precursor (APP) (Proc. Natl. Acad. Sci. USA, 87, 6003 (1991); Proc. Natl. Acad. Sci. USA, 89, 3055 (1992)), and abnormal phosphorylation of cytoskeletal proteins (Annals of the New York Academy of Sciences, 568, 198 (1989); Archives of Neurology, 42, 1097 (1985)). Measurement of the change in calpain activity was considered to provide an important information on the etiology and treatment of Alzheimer's disease, since activation of calpain not only reflects dynamic equilibrium of intracellular calcium, but directly reflects neuronal denaturation associated with increased level of intracellular calcium.

It is known that there are two isozymes, calpain I and calpain II. These enzymes require for their activation µM and mM levels of calcium concentration, respectively. Both calpains predominantly exist within cells in the form of an inactive precursor (Cell Structure and Function, 15, 1 (1990); FEBS Letters, 220, 271 (1987)). The precursor is converted into its active form in the presence of calcium through the self-digestion process which occurs at the N-terminal (Journal of Biochemistry, 90, 1787 (1981); Journal of Biochemistry, 98, 407 (1985); Biochimica et Biophysica Acta, 1078, 192 (1991)).

It is difficult to monitor in vivo action of calpain by means of in vitro measurement of the enzyme activity. This is because the precursor is activated in the course of the measurement of activity. Thus, enzyme activity to be measured is affected by unstableness of the enzyme and various intracellular suppressor or activator (Intracellular Calcium-dependent Proteolysis (CRC Press, Boston, MA), 1 (1990))
It has been shown using antibodies to calpain that calpain exists in senile plaque and a lesional portion of neurofibril (Brain Research, 558, 105 (1991); Brain Research, 561, 177 (1991)), suggesting that calpain may participate in the formation of the pathological structure characteristic to Alzheimer's disease. However, the result from direct measurement of calpain activity showed no remarkable difference between patients suffering from Alzheimer's disease and normal healthy subjects (Biomedical Research, 10, 17 (1989); Journal of Neurological Science, 102, 220 (1991); Neurobiology of Aging, 11, 425 (1990)).

The present inventors paid special attention to the amount of each of calpain I isoforms existing in post-mortem human brain, i.e., an active form and its precursor, which correspond respectively to the active form and the precursor reported on platelet and erythrocyte (Biochemical Journal, 246, 481 (1987); Biochemical Journal, 261, 1039 (1989)). This was because that since protein degradation at physiological calcium level requires the self-digestion of calpain I (Biochimica et Biophysica Acta, 1094, 249 (1991)), the ratio between the active form and the precursor of this enzyme in tissues was considered to provide an index of the in vivo function of calpain I. With this index, the inventors discovered increased level of the active form of calpain I in brain of patients suffering from Alzheimer's disease. Thus, Alzheimer's disease may be treated by administering a patient suffering from the disease an effective amount of a calpain inhibitor. The present invention is based on these findings.

Thus, the present invention provides the method of treating a subject who is suffering from, or susceptible to, Alzheimer's disease by administering said subject a calpain inhibitor.

The invention also provides a pharmaceutical formulation for prophylactic or therapeutic treatment of Alzheimer's disease, which contains as an active ingredient a calpain inhibitor.

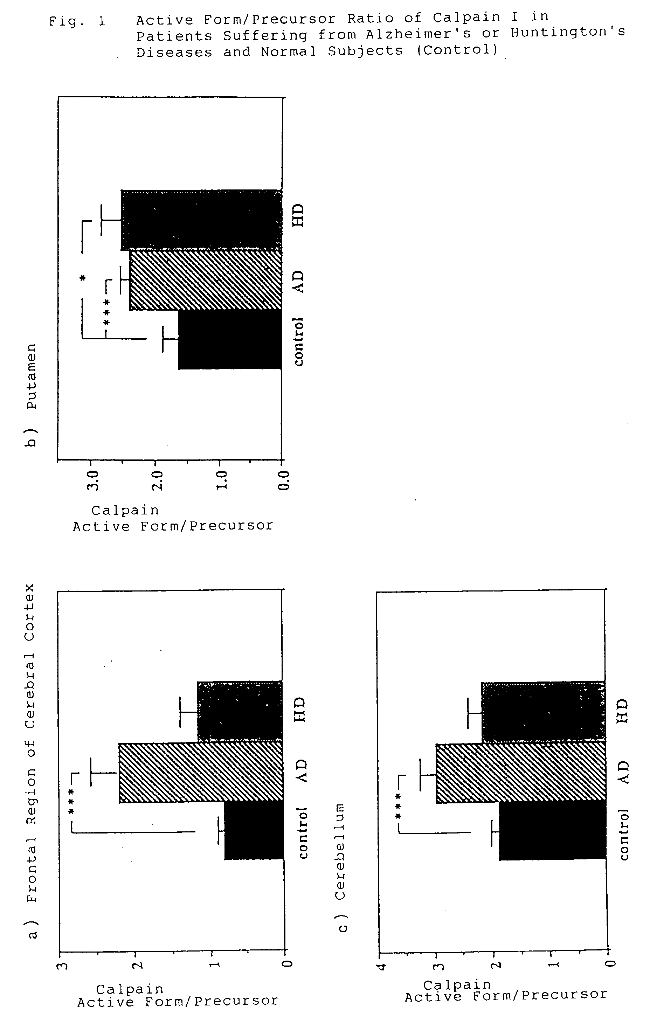

In the accompanying drawing, Fig. 1 shows the ratio of the active form of calpain I to the calpain precursor in brain of patients suffering from Alzheimer's disease (AD) or Huntington's disease (HD), or normal healthy subject (control). In the Figure, a) shows the ratio in prefrontal cortex, b) shows the ratio in putamen, and c) shows the ratio in cerebellum.

The present invention is described in more detail below.

The increased level of calpain I in brain of a patient suffering from Alzheimer's disease when compared with normal healthy subject was confirmed according to the following procedures.

Calpain I is partially purified from post-mortem brain. Fraction containing crude calpain I is subjected to electrophoresis, and reacted with monoclonal antibody to human calpain I (Biochemical Journal, 246, 481 (1987); Biochemical Journal, 261, 1039 (1989)) to show three bands. Molecular weights of these bands are 80KDa, 78KDa, and 76KDa, respectively, and are identical to those in erythrocyte (Biochemical Journal, 246, 482 (1987); Biochemical Journal, 261, 1039 (1989)). When human erythrocyte is incubated in the presence of calcium, the 80KDa band disappears and the 76KDa band appears. This reaction is inhibited by leupeptine which is a calpain inhibitor. Furthermore, there exists a good correlation between the amount of the 76KDa protein and the enzyme activity. Thus, it is believed that the 80KDa band of calpain I represents its inactive form (precursor) and the 76KDa band represents its active form. This transformation from 80KDa to 76KDa in the presence of calcium can also be observed with slice specimens of post-mortem human brain.

When the ratio between the active form and the precursor was examined in different sites of brain (prefrontal cortex, putamen, cerebellum) of a patient suffering from Alzheimer's disease, increased amounts of the active form were observed in all sites mentioned above, as described in working Example hereinafter described. This fact suggests that the increase of the active form of calpain I is not the secondary outcome of neuronal cell death, but it precedes the death of cells and may be a trigger of the death. Accordingly, an agent which inhibits the conversion of calpain I precursor to its active form, which is herein called a calpain inhibitor, is believed to prevent cells from the death and to avoid accumulation of amyloids, and therefore, to be useful for suppression or prevention of progression of Alzheimer's disease.

Examples of calpain inhibitors which may be used in the present invention include epoxysuccinic acid derivatives such as E-64 (Agricultural and Biological Chemistry, 42, 529 (1978)), E-64-C (Journal of Biochemistry, 90, 255 (1981)), E-64-d (Journal of Biochemistry, 93, 1305 (1983)), NCO-700 (Japanese Patent Publication (kokai) No. 126879/1983), estatine A, B (The Journal of Antibiotics, 42, 1362 (1989)) and the like, each having the following formulas. 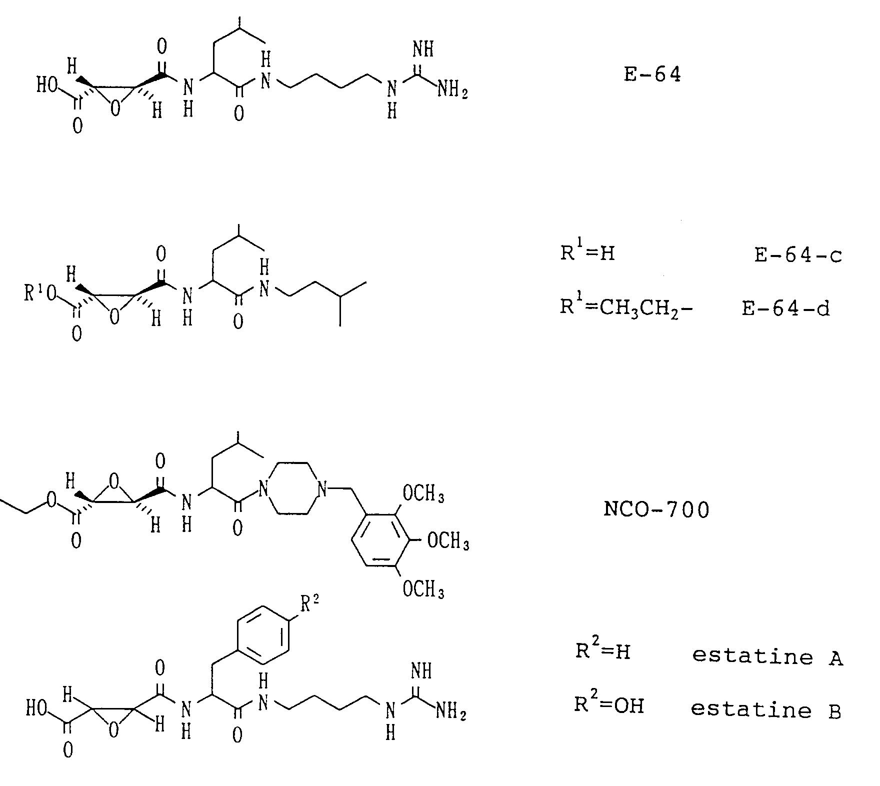
Further examples of the calpain inhibitors are peptidyl-fluoromethylketone derivatives (Journal of Medicinal Chemistry, 35, 216 (1992)), peptidyl-chloromethylketone derivatives (Journal of Biochemistry, 99, 173 (1986)), peptidyl-acyloxymethylketone derivatives (Biochemistry, 30, 4678 (1991)) having the following general formula (I): 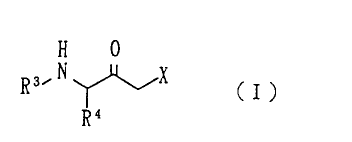
wherein R³ is an amino acid or peptide residue which may have a substituent on its N-terminal, R⁴ is an amino acid side chain-forming group, X is a fluorine atom, a chlorine atom, or a group -O-CO-Ar in which Ar is a phenyl group which may be substituted, peptidyl-diazomethylketone derivatives (Biochemical Journal, 253, 751 (1988)) having the following general formula (II): 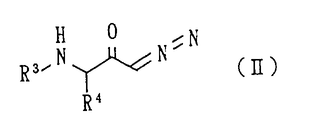
wherein R³ and R⁴ are as defined above in the general formula (I), peptidyl aldehyde derivatives such as leupeptine (The Journal of Antibiotics, 22, 183 (1969)), calpeptin (Journal of Enzyme Inhibition, 3, 195 (1990)), and MDL 28170 (Biochemical and Biophysical Research Communication, 157, 1117 (1988)), each having the following formulas. 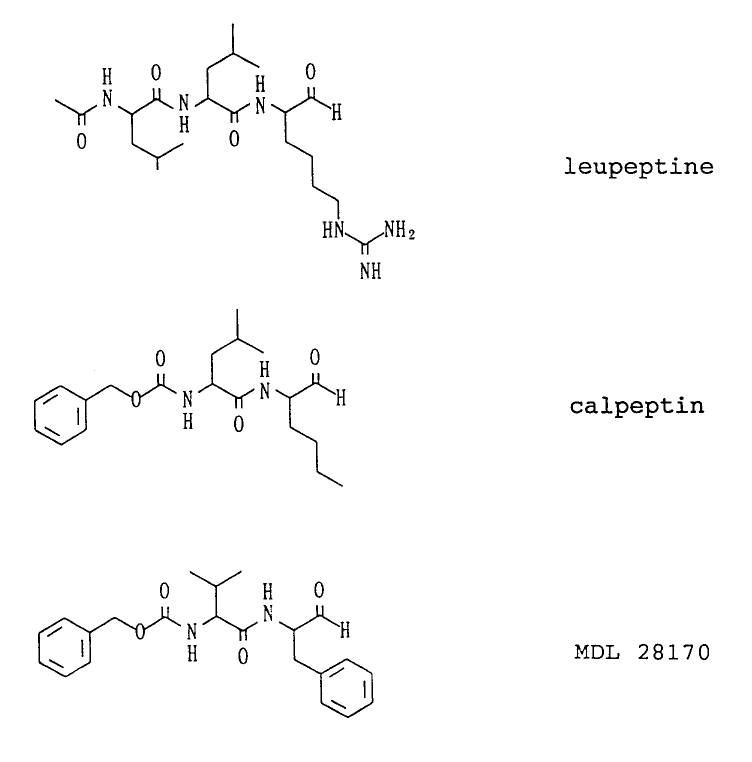
Additional examples of the calpain inhibitors are peptidyl-α-diketone or peptidyl-α-ketoester derivatives (Journal of Medicinal Chemistry, 33, 11 (1990)) having the following general formula (III): 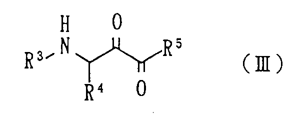
wherein R³ and R⁴ are as defined above in the general formula (I), and R⁵ is an alkyl or alkoxy group. Peptide compounds such as human brain calpastatin (U.S. Patent Application Ser. No. 200,141) or derivatives thereof are also included.

The compounds described above may be readily prepared using the methods described in the above mentioned references.

In the clinical application of the above compound, it may be administered in the form of a pharmaceutical formulation, in which the ratio of the compound to carriers may vary from 1% by weight to 90% by weight. For example, the compound may be orally administered in the form of granules, fine granules, powders, tablets, hard capsules, soft capsules, syrups, emulsions, suspensions, solutions or the like, or may be administered intravenously, intramuscularly, subcutaneously, intramedullarly, or intraventicularly in the form of injections. Furthermore, the compound may be used in the form of suppositories. Alternatively, it may be formulated in powders for injections which are used in preparing injections at the time of use. For preparing the pharmaceutical formulations of the present invention, any pharmaceutically-acceptable organic or inorganic solid or liquid carriers or diluents suitable for oral, per rectum, or parenteral administration may be used. Examples of carriers to be used for preparing solid formulations are lactose, sucrose, starch, talc, cellulose, dextrin, kaolin, calcium carbonate and the like. Liquid formulations for oral administration such as emulsions, syrups, suspensions, solutions or the like contain conventional inert diluents such as water, vegetable oils, or the like. In addition to the inert diluents, the formulations may further contain adjuvants such as humectants, suspension aids, edulcorants, aromatics, coloring agents, or preservatives. The compounds may be formulated into solutions and filled into capsules made of absorbable materials such as gelatin. Examples of medium and suspending agent to be used in preparing the formulations for parenteral administration such as injections, suppositories, or the like, include water, propylene glycol, polyethylene glycol, benzyl alcohol, ethyl oleate, lecithin, and the like. Examples of base to be used for suppositories are cacao butter, emulsified cacao butter, laurin tallow, witepsol, and the like. Such formulations may be prepared using any one of conventional methods.

The clinical dose of the compound for oral administration is generally 0.01 - 1000 mg per day for adult, though it is preferable to adjust appropriately for particular case depending on the age of particular patient, conditions and symptoms of the disease to be treated. The above daily dose of the compound may be administered as a single dose or in two or three divisions at appropriate intervals or intermittently.

When the compound is administered in the form of injection, it is preferably administrated continuously or intermittently at 0.001 - 100 mg for adult.

The following Example is illustrative only and is not intended to limit the scope of the present invention in any way.

### Example 1: Measurement of calpain in brain of a patient suffering from Alzheimer's disease

About 0.5g of a post-mortal brain was removed, homogenized in 10 volumes of 20mM Tris-HCl (pH 7.4), 2mM EGTA, 1mM EDTA, 1mM benzamidine, 1mM dithiothreitol, 1mM phenylmethylsulfonyl fluoride (PMSF), and 0.32M sucrose ("Buffer A"), and the homogenized mixture was then centrifuged (15,000xg, 30min, 4°C). The supernatant was applied to DEAE-cellulose column (DE-52, Whatman: 1.0x1.5cm), and fractions were collected during the elution with a KCl concentration gradient from 0 to 3mM. The fraction eluted at 0.15M KCl was subjected to electrophoresis and transferred onto PVDF membrane (Immobilon-P, Millipore). The resulting membrane was reacted with monoclonal calpain I antibody (Biochemical Journal, 246, 481 (1987); Biochemical Journal, 261, 1039 (1989)) and analyzed according to the method described in Journal of Neurochemistry, 92, 526 (1992).

Three bands at 80KDa, 78KDa, and 76KDa were detected in the human brain extract, and they were identical to those in human erythrocyte (Biochemical Journal, 246, 481 (1987); Biochemical Journal, 261, 1039 (1989)).

When calpain I in human erythrocyte was activated in vitro by adding calcium, N-terminal of 80KDa isoform was cleaved to create 78KDa isoform and 76KDa isoform which was resulted from cleavage of Gly²⁶-Leu²⁷ site (the cleavage site was determined by separating 76KDa isoform resulted from the self-digestion using SDS-PAGE, transferring it onto PVDF membrane and then conducting amino acid sequencing of the N-terminal using Edman degradation method). The conversion of 80KDa isoform to 76KDa isoform was observed within 30 seconds. The enzyme activity was retained even after the disappearance of 80KDa isoform, and the activity showed a good correlation with the amount of 76KDa isoform. This was consistent with the previous report suggesting that 76KDa isoform is an enzymatically active form (Biochemical and Biophysical Research Communication, 123, 331 (1984); Biochemical and Biophysical Research Communication, 138, 638 (1986)).

Although human brain calpain I is considerably unstable after extraction, the conversion of 80KDa isoform to 76KDa isoform could be observed when corticocerebral sections were incubated with calcium. Using polyclonal antibodies to a synthetic peptide comprising the N-terminal 34 amino acids of the sequence reported by Imajoh et al., Biochemistry, 27, 8122 (1988), which was prepared according to the method described in Journal of Neurochemistry, 47, 1039 (1986), it was confirmed that the self-digestion of calpain I has resulted from the loss of its N-terminal region.

In order to quantitatively determine the extent of activation of calpain I in Alzheimer's brain, the three isoforms were measured by means of immunoassay (Journal of Neurochemistry, 92, 526 (1992)). Brain from patients without neuropathy was selected as a control group, and brain from patients who were diagnosed as having dementia and showed neuropathologic characteristics of Alzheimer's disease was selected as an Alzheimer group (Archives of Neurology, 42, 1097 (1985); Neurology, 41, 479 (1991)). The average ages of patients of Alzheimer group (N=22) and the control group (N=17) were 75.8±2.0 and 68.9±2.6 years old respectively when they died. The average time-intervals between patient's death and freezing of their brains were 12.2±1.3 hours and 12.0±1.8 hours for Alzheimer group and control group respectively.

The amount of 80KDa calpain I in cytoplasm of the Alzheimer group was considerably lower than that of the control group (22.7±1.5% for the control group and 37.2±2.2% for the Alzheimer group, p<0.001 under one-sided t-test). One the other hand, the amount of 76KDa calpain I, an active form of calpain generated by self-digestion was considerably larger in the Alzheimer group than in the control group (41.2±1.6% for the Alzheimer group and 26.6±2.2% for the control group, p<0.001). There was no difference in the amounts of 78KDa calpain I between the two groups.

When the ratio of 76KDa calpain I to 80KDa calpain I in each of the brain samples was measured, it was shown that the ratio in the Alzheimer group was three times larger than that in the control group (2.20±0.39 for the Alzheimer group and 0.81±0.10 for the control group, p<0.005, see Fig. 1a). The total amount of the three calpain I isoforms was identical among samples.

Then, the intracellular distribution of calpain I was studied. Frontal cortex of cerebrum was homogenized and centrifuged at 15,000xg for 30min at 4°C to obtain a supernatant comprising cytoplasm and a precipitate comprising cell membrane. The amount of each of the three isoforms of calpain I was measured by immunoassay as described above. The results showed that 27-30% of total calpain I existed in the membrane fraction both in the control group and the Alzheimer group, and that the distribution patterns of the three isoforms in the membrane fractions were nearly identical to each other. Consequently, it was confirmed that the abnormal ratio of calpain I isoforms in the cytoplasm was not reflection of their intracellular distribution. In the membrane fraction, the amount of 76KDa calpain I (40.4±1.8% for the control group and 43.1±1.7% for the Alzheimer group, N=9 for both groups) was larger than the amount of 80KDa calpain I (18.2±1.3% for the control group and 17.5±1.3% for the Alzheimer group, N=9 for both groups). This result was consistent with the hypothesis that calpain I may be activated principally on the membrane (Journal of the Biological Chemistry, 264, 18838 (1969); Biochemical and Biophysical Research Communication, 128, 331 (1985)). Degree of abnormal activation of calpain I observed in Alzheimer's brain was not changed depending on the postmortem interval. Correlation (r) between the ratio of 76KDa calpain I to 80KDa calpain I and the post-mortal period was calculated, and r=0.355 for the control group, r=0.155 for the Alzheimer group, and r=0.148 for a group comprising both groups were obtained. Thus, there was no significant correlation between the ratio and postmortem interval. Furthermore, no significant correlation was observed between the degree of activation of calpain I and the patient's age at his death.

Since it had been recognized that a considerable amount of neuron was denatured in neocortex of patients suffering from Alzheimer's disease, degree of activation of calpain I was measured at the site in which neuronal denaturation was little or not observed (Neurology, 30, 820 (1980); The Neurobiology of Aging, 51(1983)). The ratio of 76KDa calpain I to 80KDa calpain I of the patients was significantly larger both in putamen (46%, p<0.005) and cerebellum (58%, p<0.005) than those of the control group. On the other hand, in frontal region of cerebral cortex and cerebellum of patients suffering from Huntington's disease (stage III), the distribution of calpain I isoforms was normal (see Fig. la and c). However, in putamen of patients suffering from Huntington's disease in which neuronal cell death has been observed (Journal of Neuropathology and Experimental Neurology, 44, 559 (1985); Journal of Neurology, Neurosurgery and Psychiatry, 48, 422(1985)), the ratio of the active form of calpain I had been increased by 50% (p<0.05) (see Fig 1b).

Based on the largely increased level of calpain I observed in Alzheimer's neocortex and similar observation at the site in which neuronal denaturation was little, it is very likely that this calpain I activation doesn't merely reflect the last stage of neuronal degeneration, but precedes the death of cells and possibly reflects wide range of abnormal metabolism associated with the death.

### Test 1: calpain inhibitory activity

According to the method previously described (Journal of Biological Chemistry, 259, 12489 (1984)), inhibitory activity of several compounds were determined with m-calpain which was extracted and purified from rat brain by means of the method described in Journal of Biological Chemistry, 259, 3210 (1984). The results are summarized in the following table in which the data for NCO-700 (Arzneimittel Forschung/Drug Research 36, 190 (1986)) and for MDL 28170 (Biochemical and Biophysical Research Communications, 157, 1117 (1988)) are those disclosed in the references.

| calpain inhibitory activity | |
|---|---|
| E-64 | IC₅₀=0.96µM |
| NCO-700 | IC₅₀=46µM |
| leupeptine | IC₅₀=0.36µM |
| carpeptine | IC₅₀=0.046µM |
| MDL 28170 | Kᵢ=0.007µM |

### Test 2: Acute toxicity

Acute toxicity data for the compounds previously described are reported in various published literatures as follow.
(1) E-64, E-64-c, E-64-d (Japanese Patent Publication (kokoku) No. 48/1992)
   LD₅₀>2000mg/kg (po, mouse)
(2) NCO-700 (Japanese Patent Publication (kokai) No. 126879/1983)
   LD₅₀=374mg/kg (iv, mouse)
(3) estatine A, B (Japanese patent Publication (kokai) No. 76/1978)
   LD₅₀>400mg/kg (ip, mouse)

### Formulation 1: Tablet

The following ingredients were admixed using a conventional method and compressed on a conventional equipment to form a tablet.

| | |
|---|---|
| E-64 | 30mg |
| crystalline cellulose | 60mg |
| corn starch | 100mg |
| lactose | 200mg |
| magnesium stearate | 4mg |

### Formulation 2: Granule

The following ingredients were admixed using a conventional method, compressed on a conventional equipment to form tablets, grinded, and granulated. Granules of 20-50 mesh were selected.

| | |
|---|---|
| E-64 | 10g |
| lactose | 20g |
| magnesium stearate | 1g |

### Formulation 3: Soft capsule

The following ingredients were admixed using a conventional method and filled into a soft capsule.

| | |
|---|---|
| E-64 | 30mg |
| olive oil | 300mg |
| lecithin | 20mg |

### Formulation 4: Injection

Twenty mg of vitellary lecithin and 3mg of E-64 were dissolved in chloroform thoroughly and then the chloroform was removed through lyophilization. Two ml of distilled water for injections was then added to prepare a 2ml ample.

### Formulation 5: Suppository

To 2 g of heat-melted witepsol, 30mg of E-64 was added, well mixed, filled into a rectal suppository mold and cooled to obtain a suppository.

### Formulation 6

Tablets, granules, soft capsules, injections, and suppositories were obtained according to the above procedures using leupeptine or calpeptin instead of E-64.

## Claims

1. A therapeutic or prophylactic agent for treating Alzheimer's disease which comprises as an essential ingredient a calpain inhibitor.

2. A pharmaceutical composition for therapeutically or prophylactically treating Alzheimer's disease which comprises as an essential ingredient a calpain inhibitor together with a pharmaceutically acceptable carrier therefor.

3. A method of treating a subject who is suffering from, or susceptible to, Alzheimer's disease by administering said subject a calpain inhibitor.

4. Use of a calpain inhibitor for the preparation of a pharmaceutical useful in the therapeutic or prophylactic treatment of Alzheimer's disease.
